# EUROPEAN PATENT APPLICATION

(11) **EP 2 517 680 A2**
(43) Date of publication of application: **31.10.2012**
(21) Application number: 12164159.1
(22) Date of filing: 13.04.2012
(51) Int. Cl.: A61F 13/02

(54) **Stretch adhesive bandage**

(30) Priority: 26.04.2011 JP 2011098035 U; 18.11.2011 JP 2011253180
(71) Applicant: Nitto Denko Corporation, Ibaraki-shi, Osaka 567-8680 (JP)
(72) Inventor: Kikuta, Noriyuki, Ibaraki-shi, Osaka 567-8680 (JP); Hamada, Atsushi, Ibaraki-shi, Osaka 567-8680 (JP); Matsumura, Takeo, Ibaraki-shi, Osaka 567-8680 (JP); Takagi, Shigeki, Ibaraki-shi, Osaka 567-8680 (JP)
(74) Representative: Nicholls, Michael John

(57) **Abstract**

Provided is a stretch adhesive bandage having an adhesive layer 2 and a release liner 3 laminated in this order on one surface of a bandlike fabric base 1. The adhesive bandage after peeling off the release liner 3 is stretchable at least in the longitudinal direction (in the longitudinal direction and the in the width direction in the first embodiment and only in the longitudinal direction in the second embodiment). The release liner 3 has perforation lines K (K1, K2) in the longitudinal direction and in the width direction in the first embodiment, and at least perforation line K in the width direction is formed in the second embodiment, and any perforation line is broken to be a dividing line by stretching the adhesive bandage in a direction perpendicular to the travelling direction of the perforation line.

## Description

### FIELD OF THE INVENTION

The present invention relates to a stretch (elastic) adhesive bandage that can be used in the medical field, hygiene field and the like for, for example, immobilization (restraint) of part of the body, fixing of medical equipment or gauze on the skin surface, and the like.

### BACKGROUND OF THE INVENTION

In the medical field, hygiene field and the like, adhesive bandages wherein an adhesive layer is formed on a bandlike fabric base are widely used for a great variety of situations, including, for example, use for restricting movement of part of the body (what is called taping), use for fixing medical equipment (punctured catheter, tube inserted through the mouth and the like) onto the skin around the insertion site, use for protecting a lesion together with gauze and the like, and the like.
For restricting movement of the part where an adhesive bandage is adhered to or wound around, an adhesive bandage using a non-elastic bandlike fabric base is used. When the adhesion site is a bending part, curved part and the like, where the skin surface stretches and shrinks, an adhesive bandage using a stretchable bandlike fabric base (called "stretch adhesive bandage" and the like) is used, since the bandage is required to appropriately follow movements of the skin while restricting the movements of the applied part.

Stretch adhesive bandages as conventional products have an elongated band-shape like conventional adhesive tapes, are stretchable (elastic) only in the longitudinal direction, and substantially unstretchable in the width direction (direction perpendicular to the longitudinal direction) (see JP-A-62-252495).
Such stretch adhesive bandage stretchable only in the longitudinal direction preferably restrains movement of the applied part, when adhered onto the skin surface with appropriate stretch in the longitudinal direction.

On the other hand, when various medical equipments, such as catheter and drip-needle punctured into the skin and tube etc. inserted into the mouth, are to be fixed to the periphery of the insertion site (punctured opening and lips) with a stretch adhesive bandage, the stretch adhesive bandage needs to adhere along the outline surface of both the medical equipment and the skin to certainly fix the two, and moreover, needs to follow movement of the skin and mouth as appropriate.
However, observation in detail of the state of use of an adhesive bandage stretchable only in the longitudinal direction by the present inventors has clarified that substantial absence of stretchability in the width direction prevents the tape from following movements of the skin surface, mouth and the like. As a result, the adhesive bandage may detach or fall off, or an excess stress may be applied onto the skin in the direction free of stretchability, which in turn may develop rash or blister on the skin when the bandage is applied for a long time (problem caused by fixed medical equipment).

In an attempt to solve the aforementioned problems in the fixed medical equipment, therefore, the present inventors first improved stretchability and flexibility of a bandlike fabric base, constituted the fabric base such that it can expand and contract (stretchable) in both the longitudinal direction and the width direction, and made the fabric base thinner and flexible.
As a result of the improvement, the stretch adhesive bandage can be wound around a curved surface of medical equipment sticking out from the skin or mouth to fix the equipment onto the surrounding skin, while sufficiently following movement of the surrounding skin.
When the use condition of an adhesive bandage stretchable in the both directions in clinical applications was examined in detail, however, many users (those who fix and adhere an adhesive bandage) were found to have complaints about the handlability.
The basic reason that caused low handlability was that the fabric base was constituted such that it can expand and/or contract in both directions, and made thinner and flexible, which claimed body-strength (stiffness) of the adhesive bandage itself. Consequently, the adhesive bandage tends to cover the hands (frequently during wearing latex gloves) during and after peeling off a release liner.
It was also found that loss of stiffness is not the single reason but the users feel poor handlability for different aspects, since the manner of adhesion varies among the users, particularly the procedure from peeling off of a release liner to the adhesion of the tape.

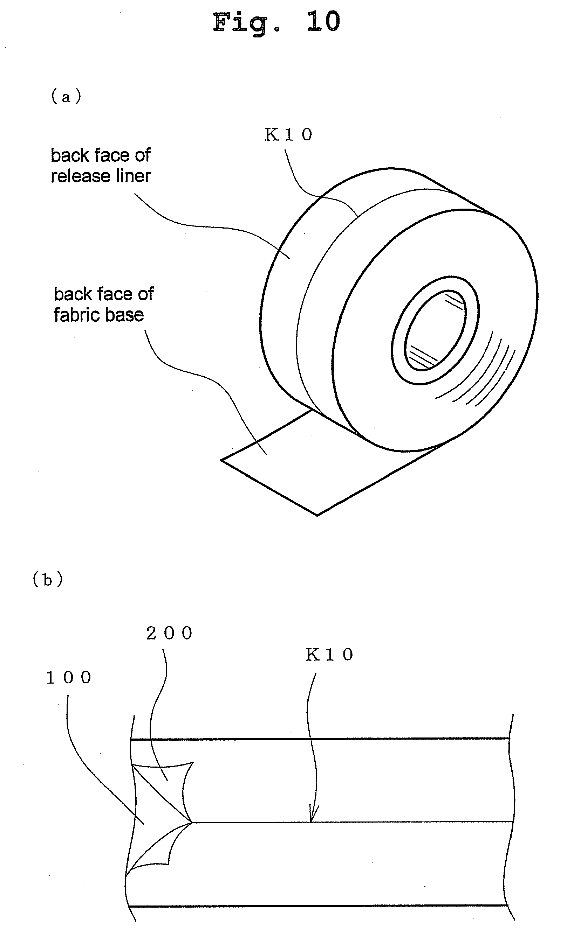

Here, a stretch adhesive bandage actually supplied for clinical practice (hereinafter to be also simply referred to as "adhesive bandage") has a conventional form shown in Fig. 10(a), wherein an elongated bandlike adhesive bandage is wound with a release liner covering the adhesive surface into a roll. The width varies depending on use.
In addition, the release liner may have one dividing line (cut line for peeling off the release liner) K10 in the longitudinal direction over the full length, as shown in Fig. 10(b), in the center of a band-shape release liner 200. In Fig. 10(b), a part of the left end of the release liner 200 is peeled off from the dividing line (cut line) K10, thus exposing the adhesive surface of an adhesive bandage 100 therebeneath.

In an attempt to clarify, in more detail, the aspects causing poor handlability felt by the users, the present inventors observed and investigated the state of use of the adhesive bandage taking note of the following (A), (B). The subject of observation was an operation to simply cover and fix to the skin a peripheral intravenous cannula (drip needle) punctured into the skin of the inner arm, with an adhesive bandage stretchable in the both directions.
(A) How users peel off a release liner by utilizing a dividing line formed in the release liner, and how they adhere the adhesive bandage to the object site.
(B) On what moment users feel poor handlability during the operation (A).

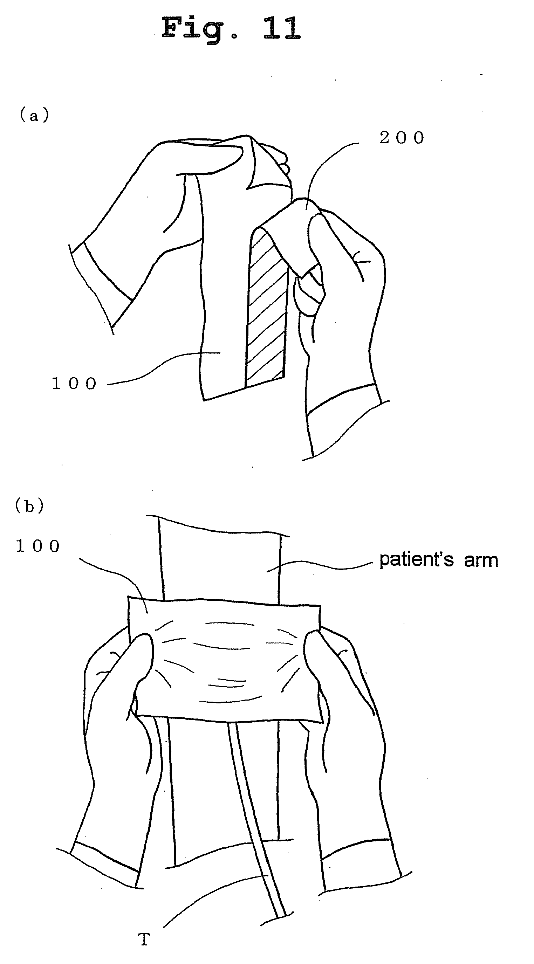

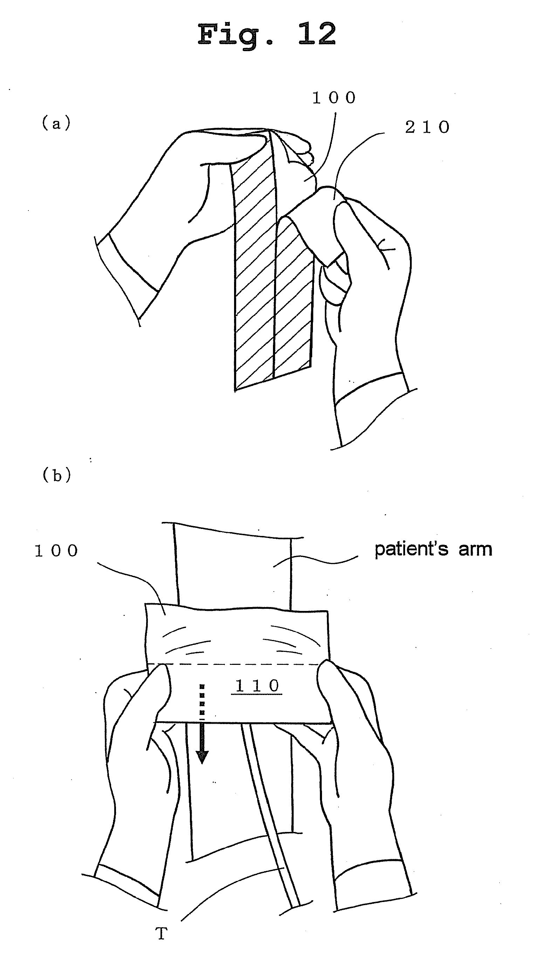

The observation and investigation by the present inventors have revealed that the above-mentioned (A) varies depending on the skill and preference of the individual but most of them showed the following procedures (a1), (a2).
(a1) As shown in Fig. 11(a), a procedure wherein a release liner 200 covering the surface of the adhesive layer is completely peeled off from adhesive bandage 100, and then, as shown in Fig. 11(b), the adhesive bandage is adhered to the whole object site (puncture site of drip needle) simultaneously or sequentially from the end by holding the both ends of the adhesive bandage with fingers of both hands. In this Figure, T shows a tube connected to a drip needle.
(a2) As shown in Fig. 12(a), a procedure wherein only one release liner 210 is peeled off from adhesive bandage 100, the exposed adhesive surface is adhered to cover half of the object site by holding the both ends of the half area 110 with the release liner, and, as shown in Fig. 12(b), the remaining half area 110 is sequentially adhered while peeling off the remaining release liner from the dividing line side towards the arrow direction. In Fig. 12(b), the dashed line drawn on the adhesive bandage is a hidden line, which shows that the release liner 210 shown in Fig. 12(a) is present on the back side (skin side) of the half area 110 of the adhesive bandage 100.

As for the above-mentioned (B), the user who employ the procedure of the above-mentioned (a1) feel poor handlability at the time of the operation to peel off the entire release liner (Fig. 11(a)), since adhesive surfaces attach to each other, they attach to unintended part of fingers and the like, which are caused by the absence of stiffness. Also at the time of adhesion (Fig. 11(b)), the absence of stiffness causes poor handlability since a thin, stiffness-free adhesive bandage covers the hands and adhesive surfaces attach to each other. However, since work steps are not complicated and efficiency is high, as well as positioning of the object site is easy, the procedure above-mentioned (a1) is preferred.
It was found that the users who employ the procedure of the above-mentioned (a1) wherein a release liner is completely peeled off often desire such operation in view of work efficiency, and the presence of the dividing line in the release liner is an obstacle to the operation, thus leading to poor handlability associated with the operation including two repeats of peeling of the release liner.
On the other hand, the users who employ the procedure of the above-mentioned (a2) wherein a release liner is present in the half area of the adhesive bandage (Fig. 12(b)) do not feel poor handlability caused by the absence of stiffness as much as for the above-mentioned procedure (a1). However, since it is difficult to refrain from touching the area without the release liner, they feel poor handlability caused by the absence of stiffness.

As mentioned above, the evaluation of poor handlability caused by the absence of stretchability in both directions and stiffness is commonly observed. However, since the original property with stretchability and stiffness is associated with the problem of poor followability to the movement of the skin and mouth, the property cannot be changed.
It was also found that the inconsistent requests of the users in favor of two repeats of peeling of the release liner as in the above-mentioned (a2) (dividing line is essential) and the users in favor of complete peeling of the release liner at once as in the above-mentioned (a1) (dividing line is obstacle) cannot be satisfied simultaneously.

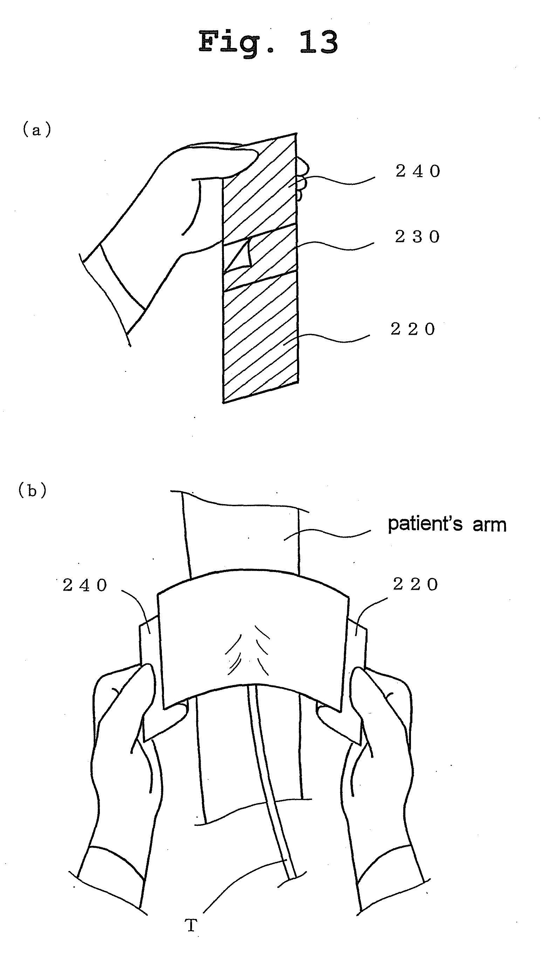

Moreover, the presence of users who desire improvement of the dividing line of a release liner, which enables procedure (a3) was also found.
(a3) As shown in Fig. 13(a), a procedure wherein a release liner is divided into three parts by two dividing lines, only the central release liner 230 is peeled off, the adhesive bandage is adhered such that the adhesive surface covers the object site with the exposed center, as shown in Fig. 13(b), and the adhesive bandage is sequentially adhered while peeling off the remaining release liners 220, 240 on both ends from the central portion side to the outer sides thereof.
Since the procedure (a3) enables adhesion of an adhesive bandage without touching the adhesive surface, wherein the adhesive bandage is increasingly adhered to the skin as the release liner is sequentially peeled off, the area free of stiffness does not increase, and therefore, poor handlability due to the absence of stiffness does not occur.
To enable the procedure (a3), two parallel dividing lines in the width direction need to be formed in a release liner, and the dividing lines (one set including two lines) need to be formed at suitable intervals throughout the entire roll of an adhesive bandage.
However, when such pairs of the dividing lines are formed, it is troublesome even for the user of the above-mentioned procedure (a3) to cut an adhesive bandage such that a pair of dividing lines comes to the center, and some portion of the adhesive bandage may be wasted.
On the other hand, when such pairs of dividing lines are set, since dividing lines increase, the handlability becomes worse for the users of the procedure (a1) above who desire to completely peel off a release liner at once. It is not preferable for the user of the above-mentioned procedure (a2), since the adhesion procedure (particularly detach direction) is different. However, when the both dividing lines are combined and dividing lines are formed in the longitudinal direction and the width direction, the handlability becomes worse for the users of the procedure (a1) above who desire to completely peel off a release liner at once. In addition, since the release liners are frequently cut at the perpendicular dividing lines, the operations of the above-mentioned (a2), (a3) cannot be performed smoothly.

As mentioned above, the problem of decreased handlability caused by making the adhesive bandage stretchable in both directions and thinner and flexible cannot be solved by restoring the stretchability of the adhesive bandage itself, and an improvement for some user made by changing the dividing lines of the release liner becomes worsening for other users. Therefore, it is extremely difficult to solve the problem in a manner any user is satisfactory as for the handlability.
This is the first problem found in the present invention.

In the present invention, an attempt is made to solve the problem caused by fixing the above-mentioned medical equipment in conventional adhesive bandages (stretchable only in the longitudinal direction) by using a more flexible bandlike fabric base.
Using a more flexible bandlike fabric base, a stretch adhesive bandage has sufficiently small stiffness, can be wound around a curved surface of medical equipment sticking out from the skin or mouth to fix the equipment onto the surrounding skin, while sufficiently following movement of the surrounding skin.
However, it was found that a flexible bandlike fabric base causes loss of stiffness of the whole adhesive bandage, and the problem of handlability of the users occurs, like the above-mentioned first problem. It was also found that the users feel poor handlability for different aspects, as in the above-mentioned first problem, since the manner of adhesion varies among the users, particularly the procedure from peeling off of a release liner to the adhesion of the tape.
This is the second problem found in the present invention.

A first object of the present invention is to provide a stretch adhesive bandage which solves the above-mentioned first problem, maintains stretchability in the longitudinal direction and the width direction, and detaches a release liner all at once or along various routes.
A second object of the present invention is to provide a stretch adhesive bandage which solves the above-mentioned second problem, and can detach a release liner completely with the smallest possible work procedures or detach and adhere a release liner by various procedures even in an embodiment showing stretchability only in the longitudinal direction.

The present inventors have conducted intensive studies in an attempt to solve the above-mentioned problems and noted the stretchability in the longitudinal direction and the width direction of the adhesive bandage, and the stretchability in the longitudinal direction of conventional adhesive bandages. They have found that, in a release liner, a perforation line formed instead of a dividing line, at least in the width direction (the longitudinal direction and the width direction in the first embodiment, the width direction in the second embodiment), which does not function as a dividing line as it is, permits the release liner to be torn at the perforation line when a user stretches the adhesive bandage in a direction perpendicular to the travelling direction of the perforation line, and turns into a dividing line (cut line), which resulted in the completion of the present invention.
Accordingly, the present invention has the following characteristics.

### SUMMARY OF THE INVENTION

(1) A stretch adhesive bandage comprising an adhesive layer and a release liner laminated in this order on one surface of a bandlike fabric base, which is stretchable at least in the longitudinal direction when a release liner is peeled off, wherein
   the release liner has one or more perforation lines at least in the width direction, and
   a selected perforation line is breakable to be a dividing line by stretching the stretch adhesive bandage in a direction perpendicular to the travelling direction of the selected perforation line.
(2) The stretch adhesive bandage of the above-mentioned (1), wherein
   the release liner has perforation lines in the longitudinal direction and the width direction, and
   a selected perforation line is breakable to be a dividing line by stretching the stretch adhesive bandage in a direction perpendicular to the travelling direction of the selected perforation line.
(3) The stretch adhesive bandage of the above-mentioned (2), wherein
   one perforation line is formed in the longitudinal direction and in the center of the release liner and
   plural perforation lines are formed in the width direction of the release liner and at equal intervals.
(4) The stretch adhesive bandage of the above-mentioned (2), wherein the interval between the plural perforation lines is 5 mm - 30 mm.
(5) The stretch adhesive bandage of the above-mentioned (2), showing a fixed load elongation each in the longitudinal direction and the width direction of 4 - 120% wherein the fixed load elongation represents stretchability of the stretch adhesive bandage when a release liner is peeled off and a load of 0.5 [N / 5 mm width] is applied in the longitudinal direction or width direction of the stretch adhesive bandage.
(6) The stretch adhesive bandage of the above-mentioned (2), wherein
   the fixed load elongation in the longitudinal direction is higher than that in the width direction,
   the fixed load elongation in the longitudinal direction is 10 - 120%, and
   the fixed load elongation in the width direction is 4 - 30%.
(7) The stretch adhesive bandage of the above-mentioned (2), wherein
   the fixed load elongation in the longitudinal direction is smaller than that in the width direction,
   the fixed load elongation in the longitudinal direction is 4 - 42%, and
   the fixed load elongation in the width direction is 22 - 120%, wherein the fixed load elongation represents stretchability of the stretch adhesive bandage when a release liner is peeled off and a load of 0.5 [N / 5 mm width] is applied in the longitudinal direction or width direction of the stretch adhesive bandage.
(8) The stretch adhesive bandage of the above-mentioned (2), wherein the bandlike fabric base is made of a knitted fabric.
(9) The stretch adhesive bandage of the above-mentioned (2), wherein the bandlike fabric base is made of a low water-absorbent fiber comprised of at least one kind selected from polyester, polyurethane, nylon, vinylon, polyethylene, polypropylene and cotton.
(10) The stretch adhesive bandage of the above-mentioned (1), which is stretchable only in the longitudinal direction when a release liner is peeled off, wherein
   the release liner has plural perforation lines at least in the width direction at predetermined intervals in the longitudinal direction, and the perforation line is broken to be a dividing line by stretching the stretch adhesive bandage in the longitudinal direction.
(11) The stretch adhesive bandage of the above-mentioned (10), wherein the perforation lines in the width direction are formed at equal intervals in the longitudinal direction of the release liner.
(12) The stretch adhesive bandage of the above-mentioned (11), wherein the interval between the perforation lines in the width direction is 5 mm - 30 mm.
(13) The stretch adhesive bandage of the above-mentioned (10), wherein a dividing line, a perforation line or a zipper line having a series of cut lines in a shape capable of facilitating the chain of division in the longitudinal direction and in the center of the release liner.
(14) The stretch adhesive bandage of the above-mentioned (10), showing a fixed load elongation of 4 - 120% wherein the fixed load elongation represents stretchability in the longitudinal direction of the stretch adhesive bandage when a release liner is peeled off and a load of 0.5 [N / 5 mm width] is applied in the longitudinal direction or width direction of the stretch adhesive bandage.
(15) The stretch adhesive bandage of the above-mentioned (10), wherein the bandlike fabric base is made of a knitted fabric knitted to have stretchability only in the longitudinal direction.
(16) The stretch adhesive bandage of the above-mentioned (10), wherein the bandlike fabric base is made of a low water-absorbent fiber comprised of at least one kind selected from polyester, polyurethane, nylon, vinylon, polyethylene, polypropylene and cotton.

### BRIEF DESCRIPTION OF THE DRAWINGS

Fig. 1 shows schematic diagrams showing the constitution of the first embodiment of the stretch adhesive bandage of the present invention, wherein Fig. 1 (a) shows a view looking straight at the outer face of the release liner and Fig. 1 (b) is a sectional view of Fig. 1 (a) along A-A.
Fig. 2 shows the manner of tearing at a perforation line formed in a release liner in the first embodiment of the stretch adhesive bandage of the present invention, wherein Fig. 2 (a) expresses, for explanation, the whole release liner like a single hard plate torn into two at the center, which is indeed torn at a perforation line on which the highest force is applied.
Fig. 3 shows views looking straight at the outer face of the release liner of the first embodiment of the stretch adhesive bandage of the present invention, which show examples of different manners of tearing and peeling off at perforation line(s) formed in the release liner, wherein in Fig. 3 (b), (c), dividing lines K1, K2e formed by tearing the release liners at the corresponding perforation lines and putting the torn pieces back in the original positions are shown with a doublet for easy review.
Fig. 4 is a view looking straight at the outer face of the release liner of the first embodiment of the stretch adhesive bandage of the present invention, which shows an example of different pattern of perforation lines formed in the release line.
Fig. 5 shows schematic diagrams showing embodiments of perforation lines by looking straight at the back face (backside of the face having release property) of the release liner of the stretch adhesive bandage of the present invention.
Fig. 6 shows schematic diagrams showing the constitution of the stretch adhesive bandage of the present invention, wherein Fig. 6 (a) shows a view looking straight at the outer face (backside of the face having release property) of the release liner and Fig. 6 (b) is a sectional view of Fig. 6 (a) along A-A.
Fig. 7 shows the manner of tearing at a perforation line formed in a release liner of the first embodiment of the stretch adhesive bandage of the present invention, wherein, in Fig. 7 (a), one perforation line K is split into two parallel lines for explanation.
Fig. 8 shows views looking straight at the outer face of the release liners of the stretch adhesive bandage of the present invention, which show examples of different manners of tearing and peeling off at a perforation line formed in the release liner, wherein in Fig. 8 (b), dividing line Ke formed by tearing the release liner at the corresponding perforation line and putting the torn pieces back in the original positions is shown with a doublet for easy review.
Fig. 9 shows a preferable embodiment of the dividing line formed in a release liner in the longitudinal direction in the present invention.
Fig. 10 schematically shows the form of a stretch adhesive bandage improved by the present inventors, and an embodiment of the dividing line in a release liner, in a stage before reaching the present invention, wherein Fig. 10 (a) is a perspective view of the form of a product of a stretch adhesive bandage to be supplied to clinical situations, and Fig. 10 (b) is a view looking straight at the outer face of the release liner, which show a dividing line in the release liner.
Fig. 11 shows one embodiment of adhesion procedures of a stretch adhesive bandage improved by the present inventors, in a stage before reaching the present invention.
Fig. 12 shows another embodiment of adhesion procedures of a stretch adhesive bandage improved by the present inventors, in a stage before reaching the present invention.
Fig. 13 shows one embodiment of adhesion procedures of a stretch adhesive bandage desired by users.

### DETAILED DESCRIPTION OF THE INVENTION

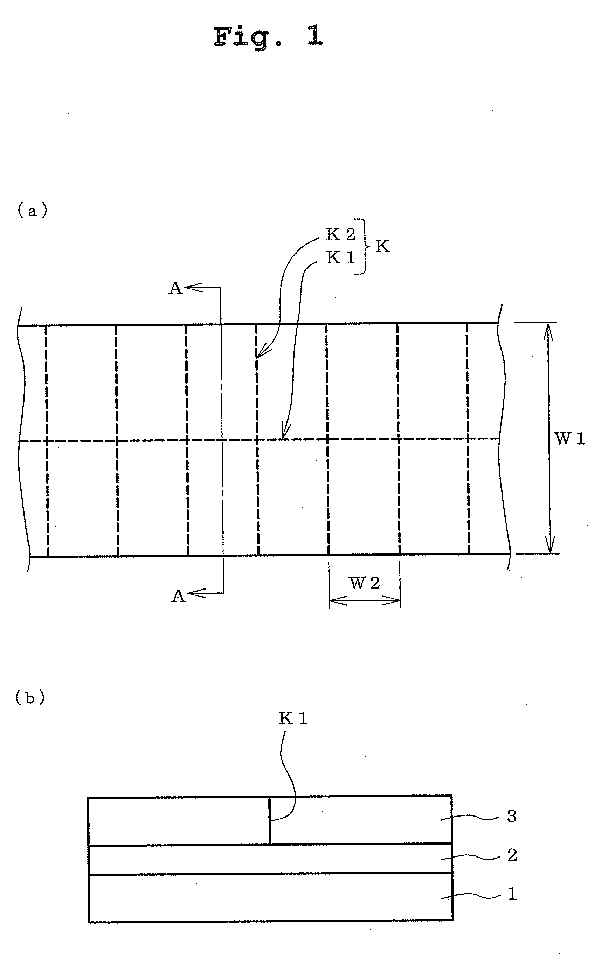

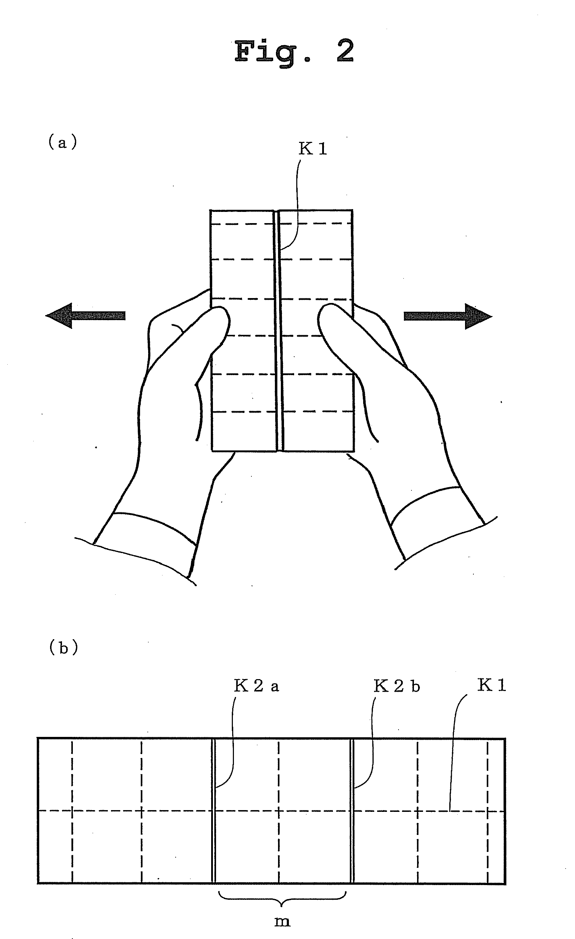

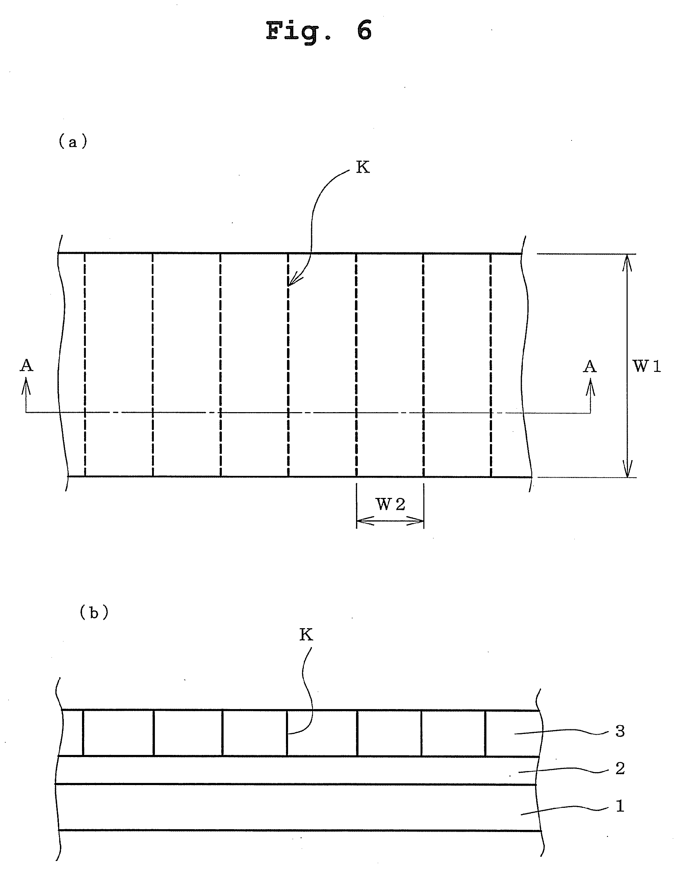

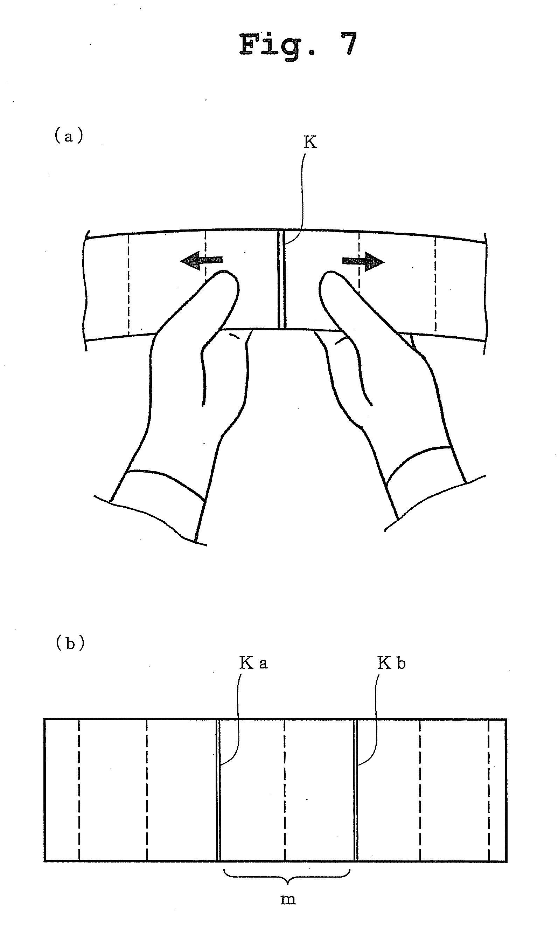

The whole constitution and constitution of each part of the stretch adhesive bandage of the present invention (hereinafter to be referred to as the adhesive bandage) are explained in detail in the following by referring to a specific embodiment.
The stretch adhesive bandage comprises, as shown in Figs. 1 and 6, an adhesive layer 2 and a release liner 3 laminated in this order on one surface of a bandlike fabric base 1, which is stretchable at least in the longitudinal direction when a release liner is peeled off. The release liner has one or more perforation lines at least in the width direction. As shown in Fig. 2 (a) and Fig. 7 (a), a selected perforation line is broken to be a dividing line by stretching the stretch adhesive bandage in a direction perpendicular to the travelling direction of the selected perforation line.
With this constitution, various manners of application of stretch adhesive bandages by the users are enabled.

### First embodiment of the present invention

In the first embodiment of the present invention, as shown in Fig. 1 (b), the adhesive bandage has a constitution wherein an adhesive layer 2 and a release liner 3 are laminated in this order on one surface of a bandlike fabric base 1. The adhesive bandage when release liner 3 is peeled off is stretchable in the longitudinal direction and the width direction, due to the stretchability imparted to bandlike fabric base 1. In the release liner 3, as shown in Fig. 1 (a), perforation line K (perforation line K1 in the longitudinal direction, perforation line K2 in the width direction) is formed in the longitudinal direction and the width direction.
As described above, since an adhesive bandage is imparted with stretchability in the longitudinal direction and the width direction, and perforation lines are formed in the longitudinal direction and the width direction, an optionally selected perforation line (in Fig. 2 (a), perforation line K1 in the longitudinal direction) is broken to be a dividing line by stretching the adhesive bandage in the direction perpendicular to the traveling direction of the perforation line (in Fig. 2 (a), width direction).

In the first embodiment of the present invention, since the stretch adhesive bandage of the present invention comprises a bandlike fabric base stretchable in the both directions of the longitudinal direction and the width direction, a medical equipment can be preferably fixed to the outer form of the skin, and the medical equipment flexibly follows the skin movement. As a result, the development of skin disorders such as rash, blister and the like can be reduced.
Furthermore, when medical equipments such as catheter, tube inserted via mouth and the like are to be fixed, they can not only be certainly fixed but also can expand, contract and follow as appropriate following the movement of the skin surface and mouth. Thus, a superior effect of resistance to detachment and falling off of adhesive bandage can be achieved.
In addition, since a fabric base is used, the stretch adhesive bandage is superior in air permeability, and does not cause skin disorder easily. Particularly, when a bandlike fabric base made of a low water-absorbent fiber is used, the stretch adhesive bandage has water repellency and can protect lesion that easily gets wet.

Next, in the first embodiment of the present invention, the property of an adhesive bandage stretchable in both directions is utilized and perforation lines in the longitudinal direction and the width direction are formed in a release liner instead of the dividing lines. As a result, various inconsistent requests of the users can be simultaneously satisfied. That is,
(A) using a perforation line rather than a dividing line, the release liner is not partially split, and therefore, the users of the procedure (a1) above can completely peel off the whole release liner at once.
(B) Users of the procedure (a2) above can perform the adhesion operation shown in Fig. 12 (a) - Fig. 12 (b), since the release liner is torn at the perforation line which becomes a single dividing line, as shown in Fig. 2 (a) wherein the perforation line is K1 in the longitudinal direction, by stretching the adhesive bandage in the arrow direction (width direction) perpendicular to the travelling direction of the perforation line.
   In addition, when a release liner is entirely peeled off from an end as in the above-mentioned (A), it can be peeled off easily by regionally stretching a suitable end of a perforation line to make a dividing line, from which the peeling is started.
(C) Moreover, as shown in Fig. 2(b), only the central portion of the release liner of Fig. 2(b) can be easily peeled off by stretching the adhesive bandage as in (B) to tear the release liner at suitable two perforation lines K2a, K2b selected from the perforation lines formed in the width direction. By this operation, an adhesion operation shown in Fig. 13(a) - Fig. 13(b) can be performed in the above-mentioned procedure (a3).

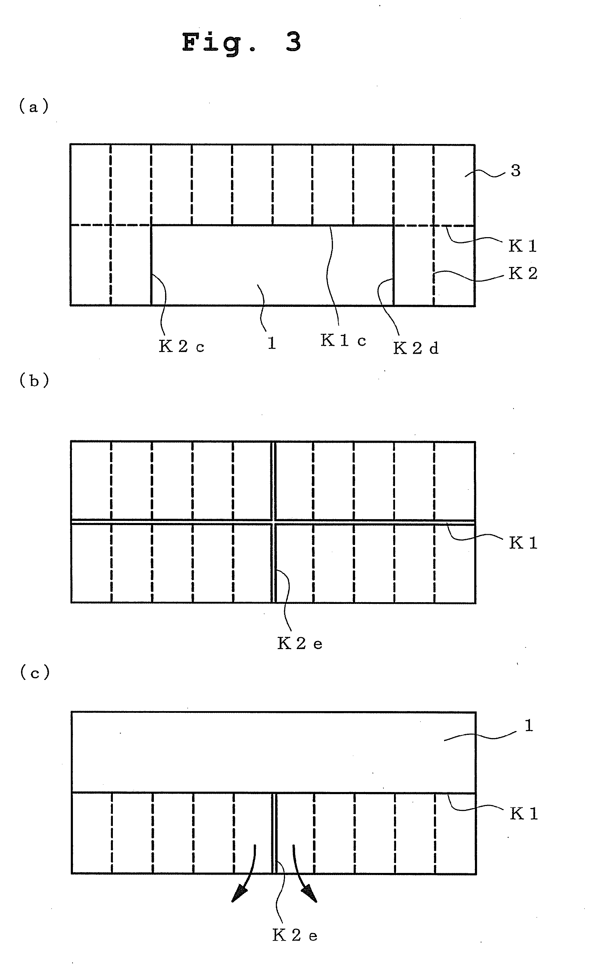

As shown in Fig. 3 (a), moreover, a release liner can have a U-shape by tearing the release liner at two perforation lines K2c, K2d in the width direction, which are far separated from each other, to the middle of K2c, K2d, tearing the release liner at perforation line K1c in the longitudinal direction and only the distance between the two perforation lines K2c, K2d, and peeling off the part of the release liner surrounded by these perforation lines. As a result, even in the absence of stiffness in the adhesive bandage, the release liner with stiffness surrounding on 3 sides like a frame facilitates handling and adhesion operation.

Furthermore, as shown in Fig. 3 (b), a release liner is torn at perforation line K1 in the longitudinal direction and then at central perforation line K2e in the width direction, and a half of the release liner is peeled off in the longitudinal direction along perforation line K1 as shown in Fig. 3 (c), thus enabling application of the procedure shown in Fig. 12 (b). The remaining half of the release liner is peeled off toward the directions shown by arrows in Fig. 3 (c) from the division line K2e toward the both sides, thus enabling application of a combination of the procedures shown in Fig. 12 (b) and Fig. 13 (b).
Such procedure not only improves the workability when applying comparatively large tubes such as drain etc. and gauze on the skin, but also advantageously reduces physical irritation to the skin since the adhesive bandage is adhered without stretching and a stress does not remain in the adhesive bandage, even when it is highly stretchable.

As mentioned above, even though the adhesive bandage of the first embodiment of the present invention is stretchable in both perpendicular directions, it can simultaneously satisfy inconsistent requests and additional requests of the users by using the perforation lines formed in a release liner, where preferable handling property conventionally unattainable can be afforded depending on how to break the perforation lines.

The adhesive bandage has a band-shaped form as a whole, like conventional adhesive tapes. Accordingly, as shown in Fig. 10 (a), a roll form is compact and preferable for provision to users.
The width and total length of the adhesive bandage are not particularly limited and may be in compliance with the users' requests. For example, the width (size W1 in Fig. 1(a)) is most often about 12 mm - 100 mm. It is convenient to provide adhesive bandages having a width varying stepwisely, for example, 12.5 mm, 25 mm, 50 mm, 75 mm, 100 mm and the like, for users' selection. In addition, since a longer total length means a larger diameter of a roll, it is suitably, for example, about 3 m - 10 m.

As a bandlike fabric base, a conventionally-known fiber usable for adhesive bandage can be used, as far as it is formed to have stretchability in the longitudinal direction and the width direction. The stretchability of an adhesive bandage after peeling off a release liner is mostly determined by the stretchability of the bandlike fabric base.
While a cloth material usable as a bandlike fabric base is not particularly limited, woven fabric, knitted fabric, non-woven fabric and the like can be used, and a knitted fabric is preferably used to impart highly recoverable stretchability in the longitudinal direction and the width direction.
Examples of the knitted fabric include warp knitting such as tricot knitting, raschel knitting and milanese stitch, and weft knitting including flat knitting and circular knitting.
To satisfy the below-mentioned stretchability (the below-mentioned "fixed load elongation") of the adhesive bandage in the longitudinal direction and the width direction, the stretchability of the bandlike fabric base in the longitudinal direction and the width direction only needs to be adjusted, specifically based on the manner of weaving longitudinal fibers and horizontal fibers, the manner of knitting, the number of stranded fibers, stranding of elastic fiber and the like.

Respective materials of warp yarn constituting a band-like fabric base (yarn woven or knitted so as to control mechanical characteristic in the longitudinal direction of the band-like fabric base) and woof yarn (yarn woven or knitted so as to control mechanical characteristic in the width direction of the band-like fabric base) are not particularly limited, and fibers made of polyester, polyurethane, nylon, vinylon, polyethylene, polypropylene, cotton and the like can be used.
Of the aforementioned materials, polyester, nylon, vinylon, polyethylene and polypropylene, which are low water-absorbent fibers, are preferably used to make the adhesive bandage water repellent. Particularly, a polyester fiber is preferably used, since it shows low water absorption and high mechanical strength.

The warp yarn and woof yarn for the band-like fabric base, and fiber as a knitting yarn preferably has a width of 100 denier or less, preferably 50 denier or less, from the aspects of breathability, stretchability, and the like of the fabric base, and 5 denier or more, from the aspects of mechanical strength to be maintained, suitable moisture transport ability, adhesion between the fabric base and adhesive layer, and the like.
While the thickness of the band-like fabric base is not particularly limited, it is preferably 10 - 150 µm, more preferably 20 - 75 µm, from the aspects of adhesion operability and prevention of uncomfortable feeling.

The stretchability of the adhesive bandage can be defined by fixed load elongation [((amount of elongation from original length ΔL)/(original length L)) x 100] when a release liner is peeled off and a load of 0.5 [N / 5 mm width] (i.e., 0.5 [N] load per 5 mm width) is applied in the longitudinal direction or width direction, which is the measurement direction, of the stretch adhesive bandage. Hereinafter a fixed load elongation measured under such conditions is to be simply referred to as "fixed load elongation".
The fixed load elongation can be obtained by the following measurement method.
(i) An adhesive bandage after removal of a release liner is cut out into rectangle test pieces (length 100 mm, width 5 mm), including a test piece having the longitudinal direction parallel to that of the adhesive bandage (test piece for longitudinal direction) and a test piece having the width direction parallel to the longitudinal direction of the adhesive bandage (test piece for width direction).
(ii) The test pieces are fixed in a tensile testing machine at distance between chucks 30 mm.
(iii) The test pieces are stretched at 300 mm/min until breakage, load and elongation amount at that time point are measured, and S-S curve (Stress-Strain curve) is obtained. The test piece for longitudinal direction and the test piece for width direction are tested 3 times each.
(iv) From the S-S curves obtained for the test piece for longitudinal direction and the test piece for width direction, the elongation rate at 0.5 [N / 5 mm width] is calculated each for the longitudinal direction and the width direction.

The stretchability in both directions of the adhesive bandage of the present invention not only improves adhesion to the skin and medical equipment, but is an essential requirement to break a perforation line of a release liner into a dividing line by stretching the adhesive bandage.
While the least stretchability necessary for breaking a perforation line of a release liner into a dividing line varies depending on the level of elongation necessary for tearing a release liner at a perforation line, the below-mentioned materials (mainly paper) for release liners break with a trace amount of elongation. Therefore, a release liner can be torn at a perforation line when stretchability in a fixed load elongation of not less than 4% can be ensured in the longitudinal direction and the width direction of an adhesive bandage.

The stretchability imparted to an adhesive bandage to improve adhesion to the skin and medical equipment is not single most preferable stretchability, and plural kinds of preferable stretchability are present for each object of use, as shown in the following embodiments (a)-(c).
(a) An embodiment wherein stretchability in the longitudinal direction and the width direction are both within a given range.
(b) An embodiment wherein stretchability in the longitudinal direction is greater than that in the width direction.
(c) An embodiment wherein stretchability in the longitudinal direction is smaller than that in the width direction.

In the embodiment of the above-mentioned (a), the levels of the fixed load elongation in the longitudinal direction and the width direction are not particularly questioned, but when the fixed load elongation in the both directions is 4% - 120%, more preferably 40% - 120%, particularly 70% - 120%, the effects of good followability to the concave convex on the skin surface and surface of medical equipment to provide close adhesion to the both surfaces. In addition, the adhesive bandage after application follows the movement of the skin and the like without an uncomfortable feeling and reduces skin irritation during the application.

In a preferable example of the above-mentioned embodiment (b), the fixed load elongation in the longitudinal direction is 10% - 120%, and the fixed load elongation in the width direction is smaller than the value. In this case, when a long adhesive bandage is adhered over a joint, a tension between the skin and the adhesive surface, which is caused by a big movement of the joint, is easily reduced, since the bandage is highly stretchable in the longitudinal direction, thus reducing a burden on the skin. In addition, since expansion and/or contraction in the width direction are/is small, when an adhesive bandage is used in a narrower width such as tracheal intubation, nasal intubation and the like, the width variation is small since expansion and contraction of the adhesive bandage in the width direction are small, which advantageously facilitates winding of the adhesive bandage around a tube.
In this case, the lower limit of the fixed load elongation in the width direction may be any as long as the perforation line can be broken to be a single line.
A preferable combination of the fixed load elongation in the longitudinal direction and the fixed load elongation in the width direction is [longitudinal direction (10% - 120%), width direction (4% - 30%)], and a more preferable combination is [longitudinal direction (40% - 120%), width direction (4% - 10%)].

In a preferable example of the above-mentioned embodiment (c), the fixed load elongation in the width direction is 22% - 120%, and the fixed load elongation in the longitudinal direction is smaller than the value. In this case, the performance of fixing medical equipment on the skin is improved. That is, a higher fixed load elongation enhances followability of an adhesive bandage to the skin, whereas it increases the risk of decreased medical equipment-fixing property, since the adhesive bandage may be elongated due to the load. Therefore, when the stretchability of an adhesive bandage has anisotropy and, for example, the fixed load elongation in the longitudinal direction is set smaller than that in the width direction, and an adhesive bandage is applied such that the longitudinal direction thereof is the same as the direction on which the load of the equipment is applied, the medical equipment can be maintained to be fixed thereon. When an adhesive bandage is peeled off from a release liner, users tend to detach the adhesive bandage at once in the longitudinal direction. In this case, when stretchability in the longitudinal direction is higher, elasticity contraction produced at the moment when the adhesive bandage is released from the release liner becomes high, thus increasing the possibility of adhesion of adhesive surfaces to each other. By setting the fixed load elongation in the longitudinal direction smaller than that in the width direction, elasticity contraction of the adhesive bandage upon detachment of the release liner becomes smaller, which advantageously avoids unnecessary adhesion of the adhesive surfaces.
In this case, the lower limit of the fixed load elongation in the longitudinal direction may be any as long as the perforation line can be broken to be a single line.
A preferable combination of the fixed load elongation in the longitudinal direction and the fixed load elongation in the width direction is [longitudinal direction (4% - 42%), width direction (22% - 120%)], and a more preferable combination is [longitudinal direction (10% - 30%), width direction (22% - 80%)].
In this case, the elongation percentage in the width direction is preferably higher than that in the longitudinal direction when tearing a release liner, rather than under fixed load conditions, and the breaking elongation (maximum elongation) obtained during the measurement of the fixed load elongation is preferably adjusted to 200 - 600% in the width direction and about 100 - 200% in the longitudinal direction.
The breaking strength is preferably adjusted to 5 - 50 [N / 5 mm width] in the width direction and 5 - 50 [N / 5 mm width] in the longitudinal direction, from the aspects of durability to load applied to the adhesive bandage when in use and operability.

The adhesive layer may be similar to the adhesive layer of the conventionally-known adhesive bandages. It preferably has an adhesive strength to adhere to medical equipment and skin surface, as well as stretchability and adhesive strength to follow, without delamination, stretchability of bandlike fabric base and movement of the skin surface.
To be specific, the adhesive layer desirably shows an adhesive strength of 2 - 10 [N/20 mm width], preferably 3 - 6[N/20 mm width], in a 180 degree peel strength test of a bakelite plate at room temperature according to the method defined in JIS Z0237.
While an adhesive showing such adhesive strength is not particularly limited, acrylic adhesive, natural rubber adhesive, synthetic rubber adhesive, silicone adhesive, vinyl ether adhesive and the like can be used. Of these, acrylic adhesive is preferably used, since adhesive property can be easily adjusted, it is less skin irritative and the like.
Specifically, an acrylic adhesive obtained by copolymerizing (meth)acrylic acid alkyl ester as a main monomer component with a monomer copolymerizable therewith, and applying, as necessary, chemical crosslinking with various crosslinking agents or physical crosslinking by irradiation of electron beam, UV and the like is preferably used.

As the main monomer component, (meth)acrylic acid alkyl ester, (meth)acrylic acid alkyl ester having an alkyl group having a carbon number of 2 - 12 can be used within the range of 40 - 99 weight%, preferably 40 - 60 weight%. As a copolymerizable monomer, one or more kinds selected from carboxyl group-containing monomer such as (meth)acrylic acid and the like, alkoxyl group-containing monomer such as (meth)acrylic acid 2-hydroxyethyl ester and the like, alkoxyl group-containing monomer such as (meth)acrylic acid 2-methoxyethyl ester and the like, acid amide group-containing monomer such as 2-vinylpyrrolidone and the like, vinyl acetate, amino group-containing monomer and the like can be used in the range of 1 - 60 weight% for copolymerization, according to the object.

While the thickness of the adhesive layer is preferably determined as appropriate in consideration of the followability to the skin, fixing property and the like, it is, for example, 10 - 180 µm, preferably 15 - 150 µm, more preferably about 20 - 80 µm.
As adhesive layer may be formed in a uniform thickness over the entirety of a bandlike fabric base, or in a pattern such as a dots-like, strip-like (linear or curved stripe-like), lattice-like, various patterns and the like.
When a strip-like adhesive layer is formed, each strip progress pattern only needs to ensure presence of a strip-like space between strip-like adhesive layers, which functions as a vent line. For example, linear stripe, wave-shaped stripe, triangular wave-shaped stripe, sawtooth wave-shaped stripe, trapezoidal wave-shaped stripe, a combination of these and the like are preferable patterns. Of these, wave-shaped stripe (what is called wavelike coating shape represented by sine wave stripe) is preferable from the aspects of ensured skin adhesive strength, prevention of curling of end portion and the like.

As a release liner, one generally used for medical adhesive tape to be adhered to the skin and the like can be used. Specific examples of the constitution of the release liner include quality paper, glassine, parchment paper and the like coated with, on the surface, a release agent having release property such as silicone resin, fluororesin and the like, a substrate (substrate of quality paper anchor-coated with resin, substrate of quality paper laminated with polyethylene resin and the like and the like) coated with, on the surface, a release agent having release property such as silicone resin, fluororesin and the like and the like can be mentioned.
While the thickness of the release liner is not particularly limited, since it is important in the present invention to tear a release liner along a perforation line in addition to be simply peeled off, about 80 - 120 µm is a preferable thickness.

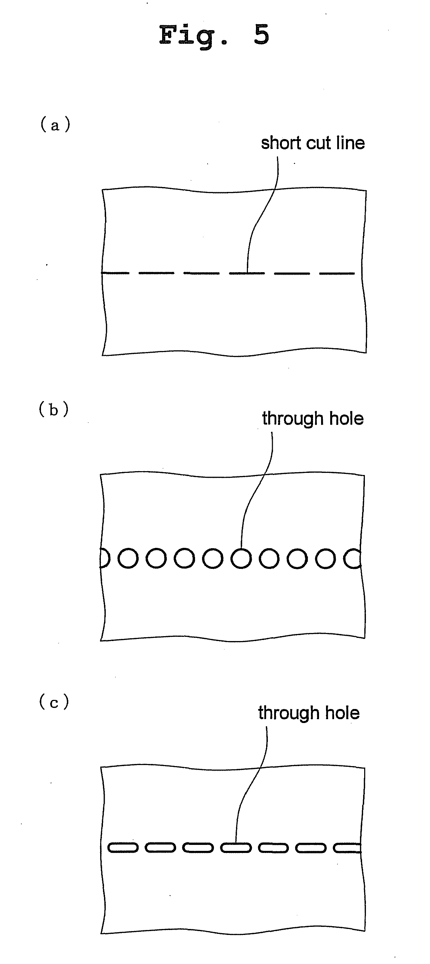

Perforations provided in the longitudinal direction and the width direction of a release liner are, as shown in Fig. 5, short cut lines and small through-holes (punched holes) arranged at regular intervals along a route intended for division, when looking straight at the back face of the release liner. The perforations may be any as long as they permit easy tearing of the release liner at its perforation line when stretched by hand in a direction perpendicular to their travelling direction. Representative embodiments of the perforation line are a dashed line and a dotted line.
In the example shown in Fig. 5 (a), the perforation line is a dashed line comprising short cut lines. Such short cut lines can be formed, for example, by partially cutting the release liner with an edged cutting tool. On the other hand, a dotted line and dashed line having round through-holes as shown in Fig. 5 (b) or elongated circular through-holes (what is called elongated hole) as shown in Fig. 5 (c) can be formed by punching out holes in a release liner.
An embodiment of a perforation line having short cut lines as shown in Fig. 5 (a) can be formed even after lamination of a release liner on an adhesive layer, by processing with an edged tool, and is easy for production. As compared to through-hole, it is a preferable embodiment, since a break heading toward the next cut line is easily induced. In the example shown in Fig. 5 (a), short cut lines are arranged to form a dashed line. The short cut lines may draw various patterns such as alternate long and short dashed lines, alternate long and two short dashed lines, repeats of particular curve and the like.
In the example shown in Fig. 5 (a), each length of the short cut lines is preferably about 0.3 mm - 10 mm, and the interval between the short cut lines (length of non-cut part) is preferably about 0.5 mm - 3 mm.
In the example shown in Fig. 5 (b), the bore diameter of the through-hole is preferably about 0.3 mm - 2 mm, and the interval between through-holes (length of part without hole) is preferably about 0.5 mm - 3 mm.
The length, interval and width of the elongated circle in the example shown in Fig. 5 (c) can be determined by referring to the length of the cut lines of Fig. 5 (a), interval between cut lines, and the bore diameter of the through-hole in Fig. 5 (b) (corresponding to width of elongated circle).

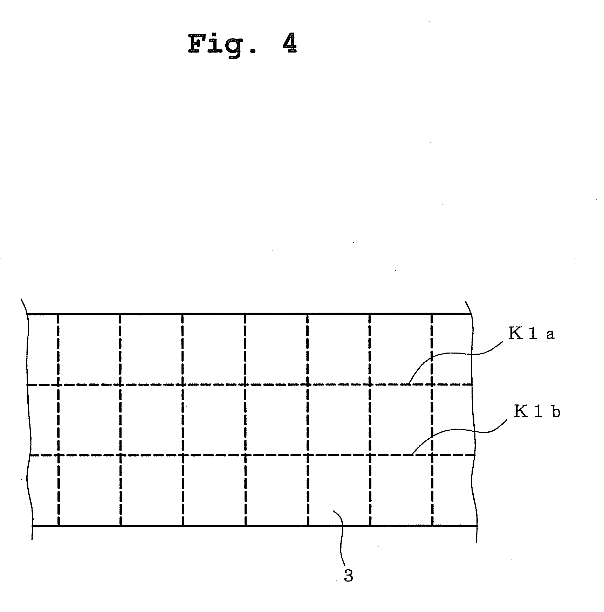

The arrangement pattern of perforation lines may be based on the number of lines and intervals meeting the users' request.
Fig. 1 (a) shows one preferable embodiment of the arrangement pattern of the perforation lines. In this example, one perforation line K1 is provided in the longitudinal direction in the position passing through the center of the width of the release liner, and plural parallel perforation lines K2 are provided at equal intervals in the width direction.
The interval W2 between perforation lines K2 in the width direction is preferably 5 mm - 30 mm, more preferably 10 mm - 25 mm, to enable peeling shown in Fig. 3 (a) and adhesion procedure shown in Fig. 13 (a) and (b).
Besides the embodiment providing one line in the center, for example, plural perforation lines at intervals of about 5 mm - 30 mm may be provided in the longitudinal direction, such as the two parallel perforation lines K1a and K1b shown in Fig. 4.

Marks and lines in the width direction are preferably printed at given intervals (e.g., 5 cm intervals and the like) on the back face of the release liner, in relation to the perforation lines in the width direction, so that it can be cut soon in a specified necessary length with scissors and the like.
In addition, when a line is printed on the perforation line, the position of the perforation line can be immediately found, and the release liner can be smoothly torn at the perforation line.

### Second embodiment of the present invention

The whole constitution and constitution of each part of the second embodiment are explained in detail in the following. In the second embodiment of the present invention, the adhesive bandage has a constitution wherein an adhesive layer 2 and a release liner 3 are laminated in this order on one surface of a bandlike fabric base 1, as shown in Fig. 6 (b). The adhesive bandage when release liner 3 is peeled off is stretchable only in the longitudinal direction, due to the stretchability imparted to bandlike fabric base 1. In the release liner 3, as shown in Fig. 6 (a), perforation line K is formed in the width direction.
As described above, since an adhesive bandage is imparted with stretchability in the longitudinal direction, and perforation lines are formed in the width direction, an optionally selected perforation line K is broken to be a dividing line by stretching the adhesive bandage in the longitudinal direction.

In the second embodiment of the present invention, since the stretch adhesive bandage of the present invention comprises a bandlike fabric base stretchable only in the longitudinal direction, and the bandlike fabric base is more flexible than conventional products, a medical equipment can be preferably fixed to the outer form of the skin, and the medical equipment flexibly follows the skin movement. As a result, the development of skin disorders such as rash, blister and the like can be reduced.
Furthermore, when medical equipments such as catheter, tube inserted via mouth and the like are to be fixed, they can not only be certainly fixed but also can expand, contract and follow as appropriate following the movement of the skin surface and mouth. Thus, a superior effect of resistance to detachment and falling off of adhesive bandage can be achieved.
In addition, since a fabric base is used, the stretch adhesive bandage is superior in air permeability, and does not cause skin disorder easily. Particularly, when a bandlike fabric base made of a low water-absorbent fiber is used, the stretch adhesive bandage has water repellency and can protect lesion that easily gets wet.

Also, in the second embodiment of the present invention, the property of an adhesive bandage stretchable in the longitudinal direction is utilized and perforation lines in the width direction are formed in a release liner instead of the dividing lines. As a result, various inconsistent requests of the users can be simultaneously satisfied. That is,
(A) forming only a perforation line in the width direction rather than a dividing line in the longitudinal direction, the release liner is not partially split, and therefore, the users of the procedure (a1) above can completely peel off the whole release liner at once.
(B) While an embodiment forming a dividing line in the longitudinal direction is also encompassed in the present invention, such embodiment requires two repeats of the step to peel off a release liner, which may be undesirable for some users. By forming a perforation line or a zipper line instead of the dividing line in the longitudinal direction, the users of the procedure (a1) above can completely peel off the whole release liner at once. In the following, a dividing line, a perforation line, and a zipper line formed along the longitudinal direction are generically referred to as a "dividing line etc.".
   Utilizing a dividing line in the longitudinal direction etc., the adhesion operation shown in Fig. 12 (a) - Fig. 12 (b) in the procedure (a2) above can be performed.
(C) Moreover, as shown in Fig. 7 (b), only the central portion of the release liner of Fig. 7 (b) can be easily peeled off by stretching the adhesive bandage as in Fig. 7 (a) in the longitudinal direction to tear the release liner at suitable two perforation lines K2a, K2b selected from the perforation lines formed in the width direction. By this operation, an adhesion operation shown in Fig. 13 (a) - Fig. 13 (b) can be performed in the above-mentioned procedure (a3).

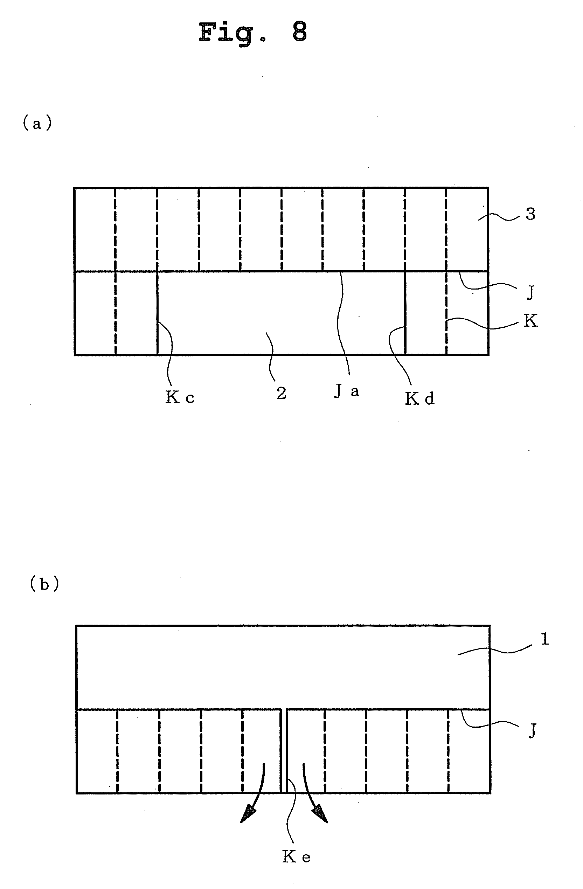

As shown in Fig. 8 (a), moreover, a release liner can have a U-shape by tearing the release liner at two perforation lines Kc, Kd in the width direction, which are far separated from each other, to the middle of Kc, Kd, tearing the release liner at dividing line etc. Ja in the longitudinal direction and only the distance between the two perforation lines Kc, Kd, and peeling off the part of the release liner surrounded by these lines. As a result, even in the absence of stiffness in the adhesive bandage, the release liner with stiffness surrounding on 3 sides like a frame facilitates handling and adhesion operation.

Furthermore, as shown in Fig. 8 (b), a release liner is torn at dividing line etc. J in the longitudinal direction and then at central perforation line Ke in the width direction, and a half (upper half in the Figure) of the release liner is peeled off in the longitudinal direction, thus enabling application of the procedure shown in Fig. 12 (b). The remaining half of the release liner is peeled off toward the directions shown by arrows in Fig. 8 (b) from the division line K2e toward the both sides, thus enabling application of a combination of the procedures shown in Fig. 12 (b) and Fig. 13 (b).
Such procedure not only improves the workability when applying comparatively large tubes such as drain etc. and gauze on the skin, but also advantageously reduces physical irritation to the skin since the adhesive bandage is adhered without stretching and a stress does not remain in the adhesive bandage, even when it is highly stretchable.

As mentioned above, even though the adhesive bandage of the second embodiment of the present invention is flexible and stretchable in the longitudinal direction, it can simultaneously satisfy inconsistent requests and additional requests of the users by using the perforation lines formed in a release liner, where preferable handling property conventionally unattainable can be afforded depending on the utilization manner of the perforation lines.

In the second embodiment of the adhesive bandage, the constitution of the following basic parts may be the same as those in the aforementioned first embodiment:
form of adhesive bandage as a whole;
cloth material usable as a bandlike fabric, materials of yarn constituting a band-like fabric base, thickness of yarn, thickness of bandlike fabric base;
measurement method of fixed load elongation that defines stretchability of the adhesive bandage;
adhesive strength, material, thickness, formation pattern of adhesive layer;
material, thickness of release liner, kind of hole of perforation line, printing on outer face of release liner (printing of cutting line as a mark).

The stretchability in the longitudinal direction of the adhesive bandage of the second embodiment of the present invention not only improves adhesion to the skin and medical equipment, but is an essential requirement to turn a perforation line in the width direction of a release liner into a dividing line by stretching the adhesive bandage.
While the least stretchability in the longitudinal direction necessary for turning a perforation line in the width direction of a release liner into a dividing line varies depending on the level of elongation necessary for tearing a release liner at a perforation line, the below-mentioned materials (mainly paper) for release liners break with a trace amount of elongation. Therefore, a release liner can be torn at a perforation line when stretchability in a fixed load elongation of not less than 4% can be ensured in the longitudinal direction of an adhesive bandage.

In the second embodiment of the present invention, the fixed load elongation in the longitudinal direction is not particularly limited, but when it is 4% - 120%, more preferably 40% - 120%, particularly 70% - 120%, the effects of good followability to the concave convex on the skin surface and surface of medical equipment to provide close adhesion to the both surfaces. In addition, the adhesive bandage after application follows the movement of the skin and the like without an uncomfortable feeling and reduces skin irritation during the application.
In addition, when the fixed load elongation in the longitudinal direction is smaller, the performance of fixing medical equipment on the skin is improved. That is, a higher fixed load elongation enhances followability of an adhesive bandage to the skin, whereas it increases the risk of decreased medical equipment-fixing property, since the adhesive bandage may be elongated due to the load. Therefore, when the fixed load elongation in the longitudinal direction is set as small as about 4 - 30% and an adhesive bandage is applied such that the longitudinal direction thereof is the same as the direction on which the load of the equipment is applied, the medical equipment can be maintained to be fixed thereon. When an adhesive bandage is peeled off from a release liner, users tend to detach the adhesive bandage at once in the longitudinal direction. In this case, when stretchability in the longitudinal direction is higher, elasticity contraction produced at the moment when the adhesive bandage is released from the release liner becomes high, thus increasing the possibility of adhesion of adhesive surfaces to each other. By setting the fixed load elongation in the longitudinal direction as small as about 4 - 30%, elasticity contraction of the adhesive bandage upon detachment of the release liner becomes smaller, which advantageously avoids unnecessary adhesion of the adhesive surfaces.

In the second embodiment of the present invention, an adhesive bandage stretchable only in the longitudinal direction means that the fixed load elongation as defined above is less than 4% relative to the width direction. For the anisotropic characteristic of [stretchable in the longitudinal direction and not stretchable in the width direction] to be marked, the two directions preferably show an unsimilar stretchability. For this reason, the fixed load elongation in the width direction is preferably not more than 3%, more preferably not more than 2%.

The breaking strength is preferably adjusted to 10 - 40 [N / 5 mm width] in the width direction and 10 - 50 [N / 5 mm width] in the longitudinal direction, from the aspects of durability to load applied to the adhesive bandage when in use and operability.

The arrangement pattern of perforation lines may be based on the number of lines and intervals meeting the users' request.
Fig. 6 (a) shows one preferable embodiment of the arrangement pattern of the perforation lines. In this example, plural parallel perforation lines K are provided at equal intervals in the width direction.
The interval W2 between perforation lines K in the width direction is preferably 5 mm - 30 mm, more preferably 10 mm - 25 mm, to enable peeling shown in Fig. 8 (a) and adhesion procedure shown in Fig. 13 (a) and (b).

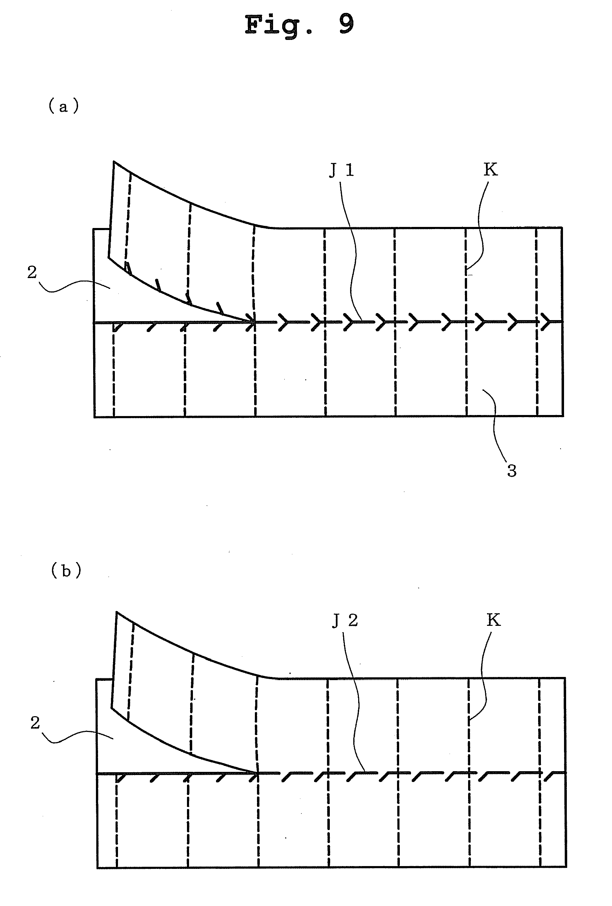

In the present invention, dividing line and perforation line may also be formed in the longitudinal direction of a release liner.
However, an embodiment having dividing line J in the longitudinal direction as shown in Fig. 8 (a), requires two repeats of the step to peel off a release liner, which may be undesirable for some users. In addition, since the stretch adhesive bandage is not stretchable in the width direction, a perforation line formed in the longitudinal direction does not permit easy tearing of a release liner by stretching the stretch adhesive in the width direction. In this case, a release liner in an area separated by a perforation line is peeled off along the perforation line to break the perforation line. However, such manner of tearing a release liner may cause deviation of the tearing direction from the perforation line, thus failing to follow the perforation line.
In contrast, a zipper line (i.e., one kind of perforation line wherein cut lines with a shape facilitating chain of divisions run in a row) as shown in Fig. 9 is preferable, since a release liner can be torn as desired. The detailed form of cut lines in a zipper line may be a known form such as those formed in the opening of, for example, paper boxes of confectionery and the like. In the constitution of Fig. 9 (a), Y-shaped cut lines run to form a dashed line and, as shown in this Figure, the release liner is torn from an end of one Y-shaped cut line as one side of the release liner is peeled off, and the break in the liner easily reaches the next Y-shaped cut line. Fig. 9 (b) shows an embodiment of L-shaped cut lines.
Besides the embodiment providing one dividing line etc. in the center, for example, plural dividing line etc. may be provided in plurality at necessary positions.

### EXAMPLES

### Example 1

### First embodiment of the present invention

In the present Examples, an adhesive bandage was actually produced, and the manner of breaking at perforation lines by utilizing stretchability in both directions was confirmed. In addition, various perforation lines were utilized to peel off a release liner and adhere an adhesive bandage, and action of perforation lines, improvement of handlability, adhesion state and the like were confirmed.

### [Kind of samples produced]

The samples of the adhesive bandage produced were the following 3 kinds having different stretchability.
(a) A sample showing a fixed load elongation in the longitudinal direction and a fixed load elongation in the width direction, which are only slightly different so that the difference between both directions is difficult to feel when stretched in the longitudinal direction and the width direction respectively. In this Example, the sample showed a fixed load elongation in the longitudinal direction of 20% and that in the width direction of 30%.
(b) A sample that can be stretched to the minimum level in the width direction. In this Example, the sample showed a fixed load elongation in the longitudinal direction of 15% and that in the width direction of 4%.
(c) A sample that can be stretched sufficiently in the width direction. In this Example, the sample showed a fixed load elongation in the longitudinal direction of 24% and that in the width direction of 120%.

### [Form of whole sample]

The form of each sample as a whole has a band shape with length 100 mm and width 50 mm.

### [Fabric base]

In the stage before processing (cutting) into a sample, a fabric base does not have a band shape but is an original rectangle fabric with length 400 mm x width 100 mm.
Fabric base for the above-mentioned sample (a): The fabric is a non-woven fabric, the material of the fiber (yarn) is polyurethane, and the thickness is 180 µm.
Fabric base for the above-mentioned sample (b): The fabric is a longitudinal elongation woven fabric, the material of the fiber (yarn) is cotton (core of warp yarn is polyurethane yarn), and the thickness is 490 µm.
Fabric base for the above-mentioned sample (c): The fabric is a knitted fabric (tubular-knitted fabric), the material of the fiber (yarn) is polyester, and the thickness is 520 µm.

### [Adhesive layer]

Material of the adhesive layer: acrylic adhesive (acrylic acid 2-ethylhexyl ester/acrylic acid copolymer)

### [Release liner]

Material and structure: paper adhered with silicone resin film with a polyethylene binder
Thickness: 98 µm

### [Perforation line]

The perforation lines formed in the release liner were those having cut lines in dashed line as shown in Fig. 5 (a) which were formed in the configuration pattern shown in Fig. 1 (a).
Each short cut line in the dashed line had length of 2.0 mm, and the part without cutting had length of 1.2 mm.
Width W2 between perforation lines formed in the width direction was 15 mm.

### [Preparation of adhesive bandage sample]

Fabric bases for the samples of the above-mentioned (a) - (c) were prepared and samples for evaluation were prepared by the following processing procedures.
(i) An adhesive solution was applied onto a release liner such that the thickness of the adhesive layer after drying was about 50 - 60 µm and dried with heating in a drying machine to give a necessary number of adhesive sheets.
(ii) The fabric base for each sample of the above-mentioned (a) - (c) was respectively adhered to the adhesive surface of each adhesive sheet obtained in the above-mentioned (i) and press-adhered by a roller to give an adhesive bandage (original fabric (whole cloth) size).
(iii) The adhesive bandage obtained in the above-mentioned (ii) was cut out in 50 mm band-shape wherein the machine direction of adhesive solution coating was the longitudinal direction.
(iv) The release liner alone was cut from the outer face of the release liner to form a perforation line with a circular cutter with a gear-blade for forming a perforation line.
(v) The adhesive bandage obtained in the above-mentioned (iv) was cut out in a given length in the longitudinal direction to give a sample for evaluation.

### [Evaluation of perforation line]

The 3 kinds of samples obtained as mentioned above were tested to determine whether the perforation line is broken. As a result, it was found that any sample including the above-mentioned sample (b) with small stretchability in the width direction permitted tearing of a release liner at a desired perforation line by stretching the adhesive bandage with both hands in a direction perpendicular to the travelling direction of each perforation line, thereby changing the perforation line into a dividing line.
It was also confirmed that, by slightly breaking the terminal portion of the perforation line, the release liner could be easily detached all at once from the broken portion.

### [Evaluation of adhesiveness utilizing perforation line]

(I) The adhesive bandage was stretched to tear a release liner at a perforation line K1 in the longitudinal direction as shown in Fig. 2 (a), the release liner was peeled off to half as shown in Fig. 12 (a), the adhesive bandage was adhered to an inner part of a lower arm, to which a catheter for drip had been temporarily held, as an apparatus model of peripheral intravenous cannula, as shown in Fig. 12 (b), and the remaining release liner was peeled off.
   In all samples, a release liner could be peeled off smoothly at a perforation line..
(II) The adhesive bandage was stretched to tear a release liner at two perforation lines K2a, K2b in the width direction as shown in Fig. 2 (b), the central release liner was peeled off as shown in Fig. 13 (a), the adhesive bandage was adhered to an inner part of a lower arm, to which a catheter for drip had been temporarily held, as an apparatus model of peripheral intravenous cannula, as shown in Fig. 13 (b), and the remaining release liner was peeled off to the both sides. In all samples, a release liner could be peeled off smoothly at a perforation line and the adhesive bandage could be applied easily.
(III) The adhesive bandage was stretched to tear a release liner at perforation lines to form a U-shape as shown in Fig. 3 (a), the rectangle part was peeled off to leave the release liner in a U-shape, the adhesive bandage was adhered to an inner part of a lower arm, to which a catheter for drip had been temporarily held, as an apparatus model of peripheral intravenous cannula, as shown in Fig. 12 (b), and the remaining release liner was peeled off.
   In all samples, a release liner could be peeled off in a U-shape smoothly at perforation lines and the remaining release liner was peeled off easily. In the adhesion operation, since the release liner with stiffness surrounded 3 sides of the adhesive bandage like a frame, handling property and adhesion workability were very good.
(IV) The adhesive bandage was stretched to tear a release liner at perforation lines in a crisscross as shown in Fig. 3 (b), the release liner was peeled off to half as shown in Fig. 3 (c), the adhesive bandage was adhered to an inner part of a lower arm, to which a catheter for drip had been temporarily held, as an apparatus model of peripheral intravenous cannula, as shown in Fig. 12 (b), and the remaining release liner was peeled off from the central perforation line K2e.
   In all samples, a release liner could be peeled off smoothly at a perforation line.

### Example 2

### Second embodiment of the present invention

In the present Examples, an adhesive bandage was actually produced, and the manner of breaking at perforation lines by utilizing stretchability in the longitudinal direction was confirmed. In addition, various perforation lines were utilized to peel off a release liner and adhere an adhesive bandage, and action of perforation lines, improvement of handlability, adhesion state and the like were confirmed.

The samples of the adhesive bandage produced showed a fixed load elongation in the longitudinal direction of 52% and that in the width direction of 3.6%.
The form of the sample as a whole has a band shape with length 100 mm and width 50 mm.

### [Fabric base]

In the stage before processing (cutting) into a sample, a fabric base does not have a band shape but is an original rectangle fabric with length 400 mm x width 100 mm.
The fabric base was longitudinal elongation and contracting woven fabric. The warp yarn has a core of urethane yarn, and is expanding and contracting yarn with cotton fiber wound therearound. The weft yarn is a cotton yarn free of stretchability with a thickness of 700 µm.

### [Adhesive layer]

Material of the adhesive layer: acrylic adhesive (acrylic acid 2-ethylhexyl ester/acrylic acid copolymer)

### [Release liner]

Material and structure: paper adhered with silicone resin film with a polyethylene binder
Thickness: 98 µm

### [Perforation line]

The perforation lines formed in the release liner are those having cut lines in dashed line as shown in Fig. 5 (a) which are formed in the configuration pattern shown in Fig. 6 (a).
Each short cut line in the dashed line had length of 2.0 mm, and the part without cutting had length of 1.2 mm.
Width W2 between perforation lines formed in the width direction was 15 mm.

### [Preparation of adhesive bandage sample]

The above-mentioned fabric bases were prepared and samples for evaluation were prepared by the following processing procedures.
(i) An adhesive solution was applied onto a release liner such that the thickness of the adhesive layer after drying was about 50 - 60 µm and dried with heating in a drying machine to give adhesive sheets.
(ii) The fabric base was adhered to the adhesive surface of the adhesive sheet obtained in the above-mentioned (i) and press-adhered by a roller to give an adhesive bandage (original fabric (whole cloth) size).
(iii) The adhesive bandage obtained in the above-mentioned (ii) was cut out in 50 mm band-shape wherein the machine direction of adhesive solution coating was the longitudinal direction.
(iv) The release liner alone was cut from the outer face of the release liner to form a perforation line with a circular cutter with a gear-blade for forming a perforation line in the width direction.
(v) The adhesive bandage obtained in the above-mentioned (iv) was cut out in a given length in the longitudinal direction to give a sample for evaluation.

### [Evaluation of perforation line]

The samples obtained as mentioned above were tested to determine whether the perforation line is broken. As a result, it was found that a release liner could be torn at a desired perforation line by stretching the adhesive bandage with both hands in the longitudinal direction, thereby changing the perforation line into a dividing line.
It was also confirmed that, by slightly breaking the terminal portion of the perforation line, the release liner could be easily detached from the broken portion.

### [Evaluation of adhesiveness utilizing perforation line]

(I) The adhesive bandage was stretched to tear a release liner at two perforation lines Ka, Kb as shown in Fig. 7 (b) by stretching the adhesive bandage as shown in Fig. 7 (a), the central release liner was peeled off as shown in Fig. 13 (a), the adhesive bandage was adhered to an inner part of a lower arm, to which a catheter for drip had been temporarily held, as an apparatus model of peripheral intravenous cannula, as shown in Fig. 13 (b), and the remaining release liner was peeled off to the both sides. In all samples, a release liner could be peeled off smoothly at a perforation line and the adhesive bandage could be applied easily.

### INDUSTRIAL APPLICABILITY

In the first embodiment of the present invention, since stretchability was imparted in both the longitudinal direction and the in the width direction, the adhesive bandage can be preferably adhered closely to a curved surface of medical equipment and the skin. In addition, since a release liner has a perforation line instead of a dividing line, the release liner can be peeled off all at once or in various manners before adhesion of an adhesive bandage, to meet the requirements of the end users.
In the second embodiment of the present invention, since a bandlike fabric base stretchable only in the longitudinal direction is more flexible than conventional products, the adhesive bandage can be preferably adhered closely to a curved surface of medical equipment and the skin. In addition, since a release liner has a perforation line in the width direction, the release liner can be peeled off all at once or in various manners before adhesion of an adhesive bandage, as in the first embodiment, to meet the requirements of the end users.

This application is based on patent application Nos. 2011-098035 and 2011-253180 filed in Japan, the contents of which are incorporated in full herein.

## Claims

1. A stretch adhesive bandage comprising an adhesive layer and a release liner laminated in this order on one surface of a bandlike fabric base, which is stretchable at least in the longitudinal direction when a release liner is peeled off, wherein
the release liner has one or more perforation lines at least in the width direction, and
a selected perforation line is broken to be a dividing line by stretching the stretch adhesive bandage in a direction perpendicular to the travelling direction of the selected perforation line.

2. The stretch adhesive bandage according to claim 1, wherein
the release liner has perforation lines in the longitudinal direction and the width direction, and
a selected perforation line is broken to be a dividing line by stretching the stretch adhesive bandage in a direction perpendicular to the travelling direction of the selected perforation line.

3. The stretch adhesive bandage according to claim 2, wherein
one perforation line is formed in the longitudinal direction and in the center of the release liner and
plural perforation lines are formed in the width direction of the release liner and at equal intervals.

4. The stretch adhesive bandage according to claim 2 or 3, wherein the interval between the plural perforation lines is 5 mm - 30 mm.

5. The stretch adhesive bandage according to any one of the preceding claims, showing a fixed load elongation each in the longitudinal direction and the width direction of 4 - 120% wherein the fixed load elongation represents stretchability of the stretch adhesive bandage when a release liner is peeled off and a load of 0.5 [N / 5 mm width] is applied in the longitudinal direction or width direction of the stretch adhesive bandage.

6. The stretch adhesive bandage according to any one of claims 1 to 4, wherein
the fixed load elongation in the longitudinal direction is higher than that in the width direction,
the fixed load elongation in the longitudinal direction is 10 - 120%, and
the fixed load elongation in the width direction is 4 - 30%.

7. The stretch adhesive bandage according to any one of claims 1 to 4, wherein
the fixed load elongation in the longitudinal direction is smaller than that in the width direction,
the fixed load elongation in the longitudinal direction is 4 - 42%, and
the fixed load elongation in the width direction is 22 - 120%, wherein the fixed load elongation represents stretchability of the stretch adhesive bandage when a release liner is peeled off and a load of 0.5 [N / 5 mm width] is applied in the longitudinal direction or width direction of the stretch adhesive bandage.

8. The stretch adhesive bandage according to any one of the preceding claims, wherein the bandlike fabric base is made of a knitted fabric.

9. The stretch adhesive bandage according to claim 1, which is stretchable only in the longitudinal direction when a release liner is peeled off, wherein
the release liner has plural perforation lines at least in the width direction at predetermined intervals in the longitudinal direction, and the perforation line is broken to be a dividing line by stretching the stretch adhesive bandage in the longitudinal direction.

10. The stretch adhesive bandage according to claim 9, wherein the perforation lines in the width direction are formed at equal intervals in the longitudinal direction of the release liner.

11. The stretch adhesive bandage according to claim 10, wherein the interval between the perforation lines in the width direction is 5 mm - 30 mm.

12. The stretch adhesive bandage according to claim 9, comprising a dividing line, a perforation line or a zipper line having a series of cut lines in a shape capable of facilitating the chain of division in the longitudinal direction and in the center of the release liner.

13. The stretch adhesive bandage according to claim 9, 10, 11 or 12, showing a fixed load elongation of 4 - 120% wherein the fixed load elongation represents stretchability in the longitudinal direction of the stretch adhesive bandage when a release liner is peeled off and a load of 0.5 [N / 5 mm width] is applied in the longitudinal direction or width direction of the stretch adhesive bandage.

14. The stretch adhesive bandage according to claim 9, 10, 11, 12 or 13, wherein the bandlike fabric base is made of a knitted fabric knitted to have stretchability only in the longitudinal direction.

15. The stretch adhesive bandage according to any one of the preceding claims, wherein the bandlike fabric base is made of a low water-absorbent fiber comprised of at least one kind selected from polyester, polyurethane, nylon, vinylon, polyethylene, polypropylene and cotton.
